# EUROPÄISCHE PATENTANMELDUNG

(11) **EP 1 083 170 A1**
(43) Veröffentlichungstag der Anmeldung: **14.03.2001**
(21) Anmeldenummer: 00117682.5
(22) Anmeldetag: 17.08.2000
(51) Int. Cl.: C07D 213/55

(54) **Verfahren zur Herstellung von D-(3'-Pyridyl)-alanin**

(30) Priorität: 08.09.1999 DE 19942812
(71) Anmelder: Degussa-Hüls Aktiengesellschaft, 60287 Frankfurt am Main (DE)
(72) Erfinder: Bommarius, Andreas, Dr., 60596 Frankfurt (DE); Schwarm, Michael, Dr., 63755 Alzenau (DE)

(57) **Zusammenfassung**

Es wird ein Verfahren beansprucht, mit dem aus D,L-(3'-Pyridyl)-methylhydantoin mittels einer (D)-spezifischen Hydantoinase N-Carbamoyl-D-(3'-pyridyl)-alanin gewonnen werden kann, das durch Reaktion mit Nitrit oder einer Carbamoylase zu (D)-(3'-Pyridyl)-alanin umgewandelt werden kann.

## Beschreibung

Die vorliegende Erfindung richtet sich auf ein Verfahren zur Herstellung von D-(3'-Pyridyl)-alanin ausgehend vom D, L-(3'-Pyridyl)-methylhydantoin mittels einer (D)-spezifischen Hydantoinase.

D-(3'-Pyridyl)-alanin findet Anwendung in verschiedenen zur Krebsbekämpfung einsetzbaren LHRH-Antagonisten, u. a. Cetrotide®.

Ein Ganzzellkatalysator-Verfahren zur Herstellung von D-(3'-Pyridyl)-alanin ist aus A.S. Bommarius, M. Kottenhahn, H. Klenk und K. Drauz, "A versatile direct route from hydantoins to D-amino acids employing a resting cell biocatalyst with D-hydantoinase and D-carbamoylase activity", in: "Microbial Reagents in Organic Synthesis", ed.: S. Servi, Kluwer Acad. Publ., Dordrecht, London, Boston, *NATO ASI Series* **1992**, *C 381*, 161-174, bekannt. Das dort beschriebene Verfahren arbeitet mit einer Biomasse aus Agrobacterium radiobacter.

Aufgabe der vorliegenden Erfindung ist es deshalb, ein weiteres Verfahren zur Herstellung von D-(3'-Pyridyl)-alanin anzugeben.

Diese Aufgabe wird gemäß Anspruch 1 gelöst, indem man D,L-(3'-Pyridyl)-methylhydantoin mit einer (D)-Hydantoinase umsetzt und anschließend das entstandene N-Carbamoyl-D-(3'-Pyridyl)-alanin entweder durch eine erneute Enzymreaktion mit Carbamoylase oder durch Versetzung mit Nitrit zum D-(3'-Pyridyl)-alanin weiterreagieren läßt.

Die weiteren Ansprüche 2 bis 5 stellen bevorzugte Ausführungsformen dar. Anspruch 6 richtet sich auf eine bevorzugte Verwendung.

Dadurch, daß man D,L-(3'-Pyridyl)-methylhydantoin mit einer (D)-Hydantoinase bei einem pH-Wert zwischen 6.0 und 11.0 und bei einer Temperatur zwischen 0 °C und 80 °C umsetzt und anschließend nach Abtrennung des Biokatalysators das entstandene N-Carbamoyl-D-(3'-pyridyl)-alanin entweder durch eine erneute Enzymreaktion mit Carbamoylase oder durch Versetzung mit Nitrit zum D-(3'-Pyridyl)-alanin weiterreagieren läßt, erhält man D-(3'-Pyridyl)-alanin in für ein technisches Verfahren sehr guten Raum/Zeit-Ausbeuten und in einer hohen Reinheit.

(D)-Hydantoinasen sind im Grunde aus a) IT 1109506, 1978; b) R. Olivieri, E. Fascetti, L. Angelini und L. Degen, Enzyme and Microbial Technology, 1979, Band 1, Seiten 201 - 204; c) H. Yamada et al., J. Ferment. Technol. 1978, 56, 484ff.; d) C. Syldatk, et al., J. Biotechnol. 1990, 14, 345ff.; e) 0. Keil, M. Schneider und J. P. Razor, Tetrahedron Asymmetry, 1995, 6, 1257 - 60; f) EP 0 643 133 A2 bekannt. In keinem Fall wurde jedoch die Umsetzung von D,L-(3'-Pyridyl)-methylhydantoin zu N-Carbamoyl-D-(3'-pyridyl)-alanin und die weitere Umsetzung zu D-(3'-Pyridyl)-alanin beschrieben.

Besonders bevorzugt sind D-Hydantoinasen, wie in den Literaturstellen e) und f) beschrieben. Ganz besonders bevorzugt wird eine (D)-Hydantoinase aus Escherichia coli im erfindungsgemäßen Verfahren verwendet.

Die verwendeten (D)-spezifischen Hydantoinasen können sowohl in löslicher Form als auch immobilisiert vorliegen.

Der pH-Wert der Reaktion liegt bevorzugt zwischen 7,5 und 10,5, besonders bevorzugt zwischen 8,5 und 10. Die Temperatur liegt vorteilhafterweise zwischen 25 °C und 60 °C, besonders bevorzugt zwischen 40 °C und 50 °C.

Das so hergestellte D-(3'-Pyridyl)-alanin kann in bioaktiyen Wirkstoffen verwendet werden, z.B. in LHRH-Antagonisten.

Die Umsetzung des D,L-(3'-Pyridyl)-methylhydantoins mit einer (D)-spezifischen Hydantoinase wird ggf. unter Zugabe von Hilfsstoffen, wie Metallionen, beispielsweise Mn²⁺-Ionen, bei konstantem pH durchgeführt. Nach dem Ende der Reaktion wird das entstandene Produkt N-Carbamoyl-D-(3'-Pyridyl)-alanin durch Feinfiltration (bei immobilisierten Enzymen) oder durch Ultrafiltration (bei löslichen Enzymen) vom Biokatalysator abgetrennt. Im nächsten Schritt wird in an sich bekannter Weise (C. Syldatk, A. Läufer, R. Müller und H. Höke, Adr. Biochem. Eng. / Biotechnol. 1990, Vol. 41, S. 29 ff. und darin zitierte Literatur) N-Carbamoyl-D-(3'-pyridyl)-alanin mit Hilfe einer D-Carbamoylase zu D-(3'-Pyridyl)-alanin umgesetzt oder N-Carbamoyl-D-(3'-pyridyl)-alanin wird in saurer wäßriger Lösung, wie beispielsweise HCl-saurer-wäßriger Lösung, bei Temperaturen zwischen 0 und 30 °C durch Zugabe von Nitrit, wie beispielsweise Natriumnitrit, entcarbamoyliert und das entstandene Produkt durch Ionenaustauschchromatographie vom Salz befreit. Das Eluat wird mit Aktivkohle geklärt, das entstandene D-(3'-Pyridyl)-alanin durch Einengen des Lösungsmittels ausgefällt und getrocknet.

Vorteilhafterweise kann im erfindungsgemäßen Verfahren sowohl D,L-(3'-Pyridyl)-methylhydantoin als auch L-(3'-Pyridyl)-methylhydantoin als Ausgangsstoff verwendet werden, da die verwendeten Hydantoine unter den Reaktionsbedingungen racemisieren, so daß zu N-Carbamoyl-(D)-(3'-pyridyl)-alanin abreagiertes D-(3'-Pyridyl)-methylhydantoin durch Racemisierung nachgebildet wird.

Es ist von Vorteil, daß bei dem erfindungsgemäßen Verfahren die Konzentration des eingesetzten Hydantoins mit bis zu 0,5 mol/L ein für ein mehrstufiges enzymatisches Verfahren äußerst hohen Wert annehmen kann, ohne daß es zu Unlöslichkeiten der Edukte bzw. intermediär gebildetem N-Carbamoyl-D-(3'-pyridyl)-alanin kommt, was den Prozeß negativ hätte beeinflussen können. Darüber hinaus ist es als überraschend zu werten, daß die Entcarbamoylierung beim erfindungsgemäßen Verfahren im wesentlichen ohne Bildung von Nebenprodukten abläuft, wo doch im Molekül ein weiteres basisches Zentrum mit dem Pyridyl-Stickstoff vorhanden ist. Offensichtlich beeinflußt dieses basische Zentrum auch die Aktivitäten der eingesetzten Biokatalysatoren nicht, was unvorhersehbar war.

### Beispiele:

### Herstellung von N-Carbamoyl-(3'-Pyridyl)alanin durch Umsetzung von (3'-Pyridyl)alanin mittels D-Hydantoinase

In 50 mL Wasser werden 5 g (26 mmol) D,L-(3'-Pyridyl)methylhydantoin gelöst und mit NaOH auf pH 8,5 eingestellt. Die Temperatur wird durch ein Wasserbad auf 50°C gehalten. Sobald eine klare Lösung vorliegt, werden 600 mg der immobilisierten D-Hydantoinase 1 (Aktivität = 210 U; 1g entspricht 350 U/g) von Roche Molecular Diagnostics zur Reaktionslösung zugesetzt. Der pH-Wert wird durch pH-Statisierung mit NaOH auf 8,5 gehalten. Der Umsatzgrad wird per HPLC bestimmt.

Innerhalb von 24 Stunden erhält man 92% Umsatz und die Reaktion ist beendet. Anschliessend wird das immobilisierte Enzym abfiltriert und die Lösung direkt in der nachfolgenden Entcarbamoylierung eingesetzt.

### Herstellung von D-(3'-Pyridyl)alanin durch Entcarbamoylierung von Carbamoyl-(3'-Pyridyl)alanin mit Natriumnitrit

Die Lösung aus der o. a. Enzymreaktion wird mittels konz. Schwefelsäure auf pH 0,0 eingestellt und auf 0 - 5°C mittels Eisbad gekühlt. Nun werden 1,97 g NaNO₂ in 30 mL Wasser gelöst (0.95 mol/L) und langsam innerhalb von 30 Minuten zugetropft. Dabei ist zu beachten, das die Temperatur nicht über 15°C ansteigt. Es wird noch eine Stunde nachgerührt, bis die Reaktion vollständig beendet ist. Der Umsatz der Entcarbamoylierung (über HPLC bestimmt) liegt bei grösser 99%.

Zur Aufarbeitung wird die Lösung auf pH 7 eingestellt, das entstandene Salz abfiltriert und durch Ionenaustauschchromatographie an stark saurem Harz von Salzen befreit. Die Aminosäure wird durch Verwendung von 5%igem Ammoniak eluiert und der Ammoniak durch Vakuumdestillation bei 45°C entfernt. Die Lösung wird fraktioniert eingeengt, die zuerst ausfallende Aminosäure abfiltriert, ggf. mit Alkohol nachgewaschen und im Vakuum getrocknet.
Ausbeute: 3.03 g (69.7% der Theorie, bez. auf Hydantoin)
Reinheit: e.e.-Wert > 99%.

## Patentansprüche

1. Verfahren zur Herstellung von D-(3'-Pyridyl)-alanin,
**dadurch gekennzeichnet,**
daß man D,L-(3'-Pyridyl)-methylhydantoin mit einer (D)-Hydantoinase bei einem pH-Wert zwischen 6.0 und 11.0 und bei einer Temperatur zwischen 0 °C und 80 °C umsetzt und anschließend nach Abtrennung des Biokatalysators das entstandene N-Carbamoyl-D-(3'-pyridyl)-alanin entweder durch eine erneute Enzymreaktion mit Carbamoylase oder durch Versetzung mit Nitrit zum D-(3'-Pyridyl)-alanin weiterreagieren läßt.

2. Verfahren nach Anspruch 1,
**dadurch gekennzeichnet,**
daß eine (D)-Hydantoinase aus Escherichia coli verwendet wird.

3. Verfahren nach Anspruch 1,
**dadurch gekennzeichnet,**
daß die Hydantoinase immobilisiert vorliegt.

4. Verfahren nach Anspruch 1,
**dadurch gekennzeichnet,**
daß der pH-Wert der Reaktion zwischen 7,5 und 10,5 liegt, besonders bevorzugt zwischen 8,5 und 10.

5. Verfahren nach Anspruch 1,
**dadurch gekennzeichnet,**
daß die Temperatur zwischen 25 °C und 60 °C liegt, besonders bevorzugt zwischen 40 °C und 50 °C.

6. Verwendung des D-(3'-Pyridyl)-alanins als Bestandteil von Peptidwirkstoffen.
